(19) Europäisches Patentamt
European Patent Office
Office européen des brevets

(11) Veröffentlichungsnummer: **0 166 242**
**B1**

(12) **EUROPÄISCHE PATENTSCHRIFT**

(45) Veröffentlichungstag der Patentschrift: **03.01.90**

(21) Anmeldenummer: **85106524.3**

(22) Anmeldetag: **28.05.85**

(51) Int. Cl.$^5$: **C 07 D233/61**, C 07 D239/26, C 07 D241/12, C 07 D295/12, D 06 P 5/06, D 06 P 5/08

(54) **Wasserlösliche kationaktive Polyelektrolyte, ihre Herstellung und ihre Verwendung.**

(30) Priorität: **28.05.84 CS 3987/84**
**07.06.84 CS 4316/84**

(43) Veröffentlichungstag der Anmeldung: **02.01.86 Patentblatt 86/01**

(45) Bekanntmachung des Hinweises auf die Patenterteilung: **03.01.90 Patentblatt 90/01**

(84) Benannte Vertragsstaaten: **CH DE GB LI**

(56) Entgegenhaltungen:
**DE-A- 3 205 647**
**GB-A- 2 094 298**
**Die Akte enthält technische Angaben, die nach dem Eingang der Anmeldung eingereicht wurden und die nicht in dieser Patentschrift enthalten sind.**

(73) Patentinhaber: **Vyzkumny ustav textilniho zuslechtováni**
**CS-544 28 Dvur Králove nad Labem (CS)**

(72) Erfinder: **Dvorsky, Drahomir, Dipl.-Ing.**
**Dvur Královè n.L**
**Dukelskych bojobniku 2103 (CS)**
Erfinder: **Cerovsky, Karel**
**Dvur Královè n.L**
**Raisova 1755 (CS)**
Erfinder: **Lukác, Jiri**
**Dvur Královè n.L**
**Dukelskych bojovniku 2103 (CS)**
Erfinder: **Socha, Jaromir, Dipl.-Ing., CSc**
**Pardubice**
**Stavbaru 154 (CS)**

(74) Vertreter: **Patentanwälte Beetz sen. - Beetz jun. Timpe - Siegfried - Schmitt-Fumian**
**Steinsdorfstrasse 10**
**D-8000 München 22 (DE)**

**EP 0 166 242 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

## Beschreibung

Die Erfindung betrifft wasserlösliche kationaktive Polyelektrolyte, die bei der Textilveredlung zur Erhöhung der Anfärbbarkeit von Cellulosefasern, zur Erhöhung der Farbechtheit und zur Vollendung der Färbung mit Reaktivfarbstoffen verwendet werden können.

Nach dem Färben oder Bedrucken ist es erforderlich, den auf den Fasern ungenügend fixierten Farbstoff und sorbierte Hilfsmittel aus den Textilmaterialien zu beseitigen; darüber hinaus muß bei einigen Farbstoffgruppen noch die Auswaschbarkeit des Farbstoffs nachträglich vermindert werden, beispielsweise durch Bildung eines Komplexes aus dem Farbstoff und einer makromolekularen Verbindung mit gegenüber dem Farbstoffmolekül ungleichnamiger Ladung.

Das Waschen von Textilmaterialien bei Textilveredlungsprozessen, besonders nach dem Färben und Bedrucken, ist ein langwieriger Vorgang, der zugleich mit einem hohen Wasser- und Energieverbrauch verbunden ist.

Das Waschen ist besonders beim Färben und Bedrucken von Cellulosematerialien mit Reaktivfarbstoffen wichtig und zugleich kompliziert. Beim Färben mit derartigen Farbstoffen reagiert bekanntlich nur ein Teil des Farbstoffs mit den Cellulosefasern. Ein relativ großer Anteil des Farbstoffs hydrolysiert und muß beseitigt werden. Die Farb- oder Druckechtheit ist daher von der Vollständigkeit des Auswaschens des hydrolysierten Farbstoffs abhängig.

Zur Lösung des Problems des Auswaschens von Reaktivfarbstoffen wurden mehrere verschiedene Wege beschritten.

Vor allem wurden solche reaktiven Systeme und Farbstoffzusammensetzungen gesucht, die mit möglichst hoher Ausbeute so auf den Fasern fixiert werden, daß während des Waschens nur ein möglichst niedriger Anteil des Farbstoffs abgetrennt werden muß.

Eine andere Lösung, die auch bereits die Auswahl der Farbstoffzusammensetzung betrifft, besteht in der Verwendung von Farbstoffen, die in hydrolysiertem Zustand möglichst gut auswaschbar sind. Weitere Möglichkeiten bestehen in der Wahl des Waschverfahrens, und zwar sowohl aus technologischer als auch aus apparativer Sicht.

Eine interessante Technologie beruht auf der Verwendung von Sorbens-Suspensionen. Ihre Anwendung ist jedoch durch ihren relativ hohen Preis eingeschränkt; es gelang ferner bisher auch noch nicht, die Färbevorrichtungen so zu modifizieren, daß die Eigenschaften der Sorbentien vollständig ausgenutzt werden können.

In Vielen Fällen werden ferner auch verschiedene kationaktive Mittel eingesetzt, die zum Schluß des Waschens angewandt werden und dazu dienen, die Reste an nicht ausgewaschenem Farbstoff zu blockieren. Dieses Verfahren ist von den oben erwähnten Möglichkeiten am wenigsten zu empfehlen, da es bei den meisten Farbstoffen zu einer Beeinträchtigung der Lichtechtheit kommt.

Bisher bekannte Mittel zur Erhöhung der Anfärbbarkeit von Cellulosefasern beruhen auf von tertiären Aminen und Epichlorhydrin abgeleiteten quartären Ammoniumverbindungen mit einer reaktionsfähigen Gruppe

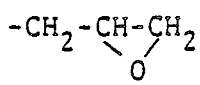 oder 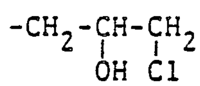 .

Höhere Wirksamkeit weisen von heterocyclischen Verbindungen mit zwei Stickstoffatomen abgeleitete quartäre Ammoniumverbindungen auf, die zwei reaktionsfähige Gruppen des obengenannten Typs enthalten. Zwei reaktive Gruppen führen zu einer entsprechend stärkeren Bindung an Cellulosefasern und vernetzen gleichzeitig die Celluloseketten (vgl. GB-A-2 094 298). Diese bekannten Verbindungen besitzen die allgemeine Formel,

$$\left[ Y - M - Y \right]^{k\oplus} \quad \frac{k}{r} X^{r\ominus}$$

in der bedeuten:

Y 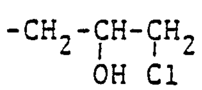 oder 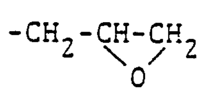 ,

r 1, 2 oder 3,
k 1 oder 2,
X ein Anion einer starken anorganischen oder organischen Säure und
M einen von einem 5- oder 6-gliedrigen Heterocyclus mit zwei Stickstoffatomen abgeleiteten

heterocyclischen Rest.

Diese Verbindungen können entsprechend zur Erhöhung der Farbechtheit der Färbung von Cellulosematerialien mit substantiven Farbstoffen verwendet werden. Ihre Wirkung bei der Färbung mit anderen Anionfarbstoffen ist jedoch nur mittelmäßig. Sie üben also einen nur kleinen Einfluß auf die Färbung mit Reaktivfarbstoffen aus und haben sich insbesondere bei der Endstufe von Ausfärbungen und Bedruckungen nicht bewährt. Auch verbessern diese vorbekannten Verbindungen das Auswaschen anionaktiver Farbstoffe nicht.

Der Erfindung liegt die Aufgabe zugrunde, neue, wasserlösliche kationaktive Polyelektrolyte, insbesondere für die oben erwähnten Einsatzzwecke, welche die Nachteile herkömmlicher Polyelektrolyte nicht aufweisen, einfach und wirtschaftlich herstellbar sind und sich beim Auswaschen von Reaktivfarbstoffen nach Färbe- oder Druckprozessen sowie auch zur Erhöhung der Farbechtheit verwenden lassen sowie ein Verfahren zu ihren Herstellung auzugeben.

Die Aufgabe wird gemäß den Patentansprüchen 1, 6 und 11 gelöst.

Die Erfindung beruht auf der überraschenden Feststellung, daß ausgeprägte Wirkungen bei der Beseitigung des hydrolysierten Anteils von Reaktivfarbstoffen von Cellulosefasern nach dem Färben oder Bedrucken und bei der Verbesserung der Farbechtheit bei Färbungen mit anderen Anionfarbstoffen, vorzugsweise mit substantiven Farbstoffen, durch Verwendung einer neuen Gruppe von kationaktiven Polyelektrolyten erzielbar sind, die erhältlich sind durch.

Umsetzung einer Verbindung der allgemeinen Formel (I),

$$\left[ Y - M - Y \right]^{k\oplus} \quad \frac{k}{r} X^{r\ominus} \qquad (I)$$

worin bedeuten :

Y —$CH_2$—CH(OH)—$CH_2$Cl oder

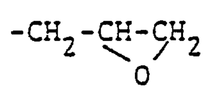 ,

M eines der bifunktionellen heterocyclischen Kationen

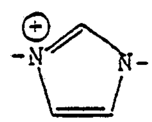 , 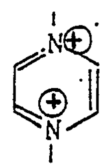 , 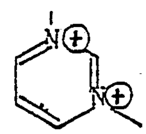 , 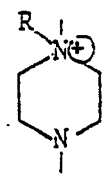

und

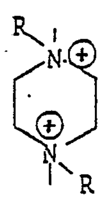 ,

wobei R jeweils H oder $C_{1-4}$-Alkyl darstellt,

X ein Anion einer starken anorganischen oder organischen Säure,

k 1 oder 2

und

r 1, 2 oder 3,

mit einem polyfunktionellen Amin der allgemeinen Formel (II),

$$H—B—H \qquad (II)$$

worin B bedeutet :

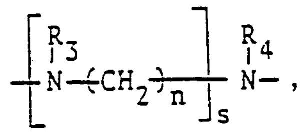

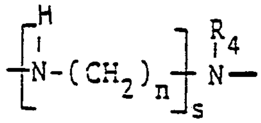 oder 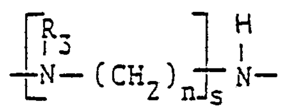

wobei $R_3$ und $R_4$ unabhängig H oder $C_{1-4}$-Alkyl,
n eine ganze Zahl von 2 bis 10 und
s 1, 2, 3 oder 4 darstellen,
in einem Molverhältnis (I) : (II) von 0,5 bis 10 bei einer Temperatur von 10 bis 100 °C.

Bei den erfindungsgemäß erhältlichen Reaktionsprodukten handelt es sich in der Regel um Gemische von Verbindungen, die durch die allgemeine Formel (III) charakterisiert werden können,

$$\left[ R_1 - A - B\,(-R_2)_x \right]^{mk\oplus} \quad \frac{mk}{r}\, X^{r\ominus} \qquad \text{(III)}$$

worin bedeuten :

A —$CH_2$—CH(OH)—$CH_2$—$M^{k\oplus}$—$CH_2$—CH(OH)—$CH_2$— ,

wobei M eines der bifunktionellen heterocyclischen Kationen

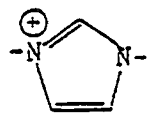 , 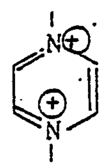 , 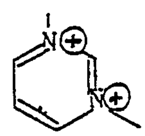 , 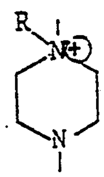

und

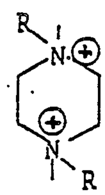 ,

mit jeweils R = H oder $C_{1-4}$-Alkyl darstellt und k gleich 1 oder 2 ist,

x 1 oder 2,
B für x = 1 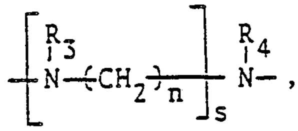

und für x = 2 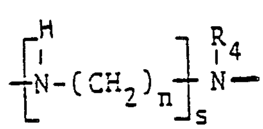 oder 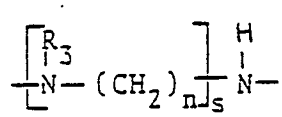

wobei

$R_3$ und $R_4$ unabhängig H oder $C_{1-4}$-Alkyl,
n eine ganze Zahl von 2 bis 10 und
s 1, 2, 3 oder 4 darstellen,
$R_1$ Cl, OH oder H-B-,
$R_2$ H, -A-Cl, -A-OH, -A-B-H, -A-B-A-OH oder -A-B-A-Cl,
X ein Anion einer starken anorganischen oder organischen Säure,
r als Wertigkeit des Anions X die Zahlenwerte 1, 2 oder 3 und
m als Anzahl der Gruppen A im Molekül die Zahlenwerte 1, 2 oder 3.

Das erfindungsgemäße Verfahren zur Herstellung der Polyelektrolyte der Formel III ist entsprechend gekennzeichnet durch

Umsetzung einer Verbindung der Allgemeinen Formel (I),

$$\left[ Y - M - Y \right]^{k \oplus} \qquad \frac{k}{r} X^{r \ominus} \qquad (I)$$

worin M, X, k, r und Y dasselbe wie oben bedeuten,
mit einem polyfunktionellen Amin der allgemeinen Formel (II)

$$H—B—H \qquad (II)$$

mit B wie oben in einem Molverhältnis (I) : (II) von 0,5 bis 10 bei einer Temperatur von 10 bis 100 °C.

Pro Mol der Verbindung der allgemeinen Formel II können entsprechend 0, 1 bis 2 mol der polyfunktionellen Amine der allgemeinen Formel II eingesetzt werden. Besonders vorteilhafte Eigenschaften Weisen die durch Umsetzung der Verbindungen (I) und (II) in einem Molverhältnis von 1 : 0,5 bis 1 : 1 erhältlichen Reaktionsprodukte auf.

Als polyfunktionelle Amine der allgemeinen Formel (II) können vorteilhaft solche handelsüblichen Verbindungen verwendet werden, die in der Epoxyharzchemie und bei der Herstellung von Komplexbildnern verwendet werden. Solche Amine sind beispielsweise Ethylendiamin, Diethylentriamin und Triethylentetramin.

Die Verbindungen der allgemeinen Formel (I) sind bereits bekannt (vgl. GB-A-2 094 298). Zur Herstellung wird üblicherweise zunächst ein Salz der entsprechenden heterocyclischen Verbindung mit einer starken Säure erzeugt, worauf eine wässerige Lösung dieses Salzes mit Epichlorhydrin so quaternisiert wird, daß auf 1 mol dieses Salzes 2 mol Epichlorhydrin entfallen. Nach Beendigung der Quaternisierung folgt das eigentliche erfindungsgemäße Verfahren, wobei das polyfunktionelle Amin der allgemeinen Formel (II) oder eine entsprechende wässerige Lösung davon zugegeben wird. Die Reaktion ist exotherm und verläuft bei einer Temperatur von 10 bis 100 °C ; sie wird vorzugsweise bei 40 bis 60 °C durchgeführt.

Nach Beendigung der Reaktion wird das Endprodukt ohne weitere Behandlungen verwendet.

Die Konzentration der aktiven Substanz in den gemischen wird durch die Handhabungsforderungen bei der Behandlung bestimmt. Es ist vorteilhaft, wässerige Lösungen der erfindungsgemäßen Reaktionsprodukte mit einer Konzentration von 40 bis 60 Masse-% anzuwenden. Lösungen höherer Konzentration weisen eine zu hohe Viskosität auf, welche die Handhabung bei der Behandlung erschwert.

Da die Verbindungen der allgemeinen Formel (I) als wässerige Lösungen mit einem Gehalt an aktiver Substanz von 70 bis 85 Masse-% anfallen, ist es zur Einhaltung der obengenannten Bedingung für die Konzentration der erfindungsgemäßen Verbindungen im Endprodukt vorteilhaft, zur Synthese die polyfunktionellen Amine der allgemeinen Formel (II) in Form von wässerigen Lösungen mit einer Konzentration von 40 bis 60 Masse-% zu verwenden.

Die resultierenden Reaktionsprodukte sind bei einem Gehalt an aktiver Substanz von 40 bis 60 Masse-% gelbbraune viskose Flüssigkeiten mit einer Dichte von 1,3 bis 1,5 g/ml, die mit Wasser in jedem Verhältnis mischbar sind.

In Tabelle 1 sind geeignete Ausgangssubstanzen und ihre Molverhältnisse bei der Reaktion angegeben.

In Tabelle 2 sind Formeln von elementaren Struktureinheiten der daraus resultierenden Verbindungen der Formel (III) angegeben.

Die erfindungsgemäßen Verbindungen der Formel (III) können auch noch endständige Epoxygruppen aufweisen.

Die erfindungsgemäßen Verbindungen bzw. Reaktionsprodukte weisen eine ganze Reihe von in der Veredlungstechnologie verwendbaren Eigenschaften auf. In alkalischen Medien können sie mit alkoholischen OH-Gruppen der Cellulosemakromoleküle reagieren und derart deren Vernetzung verursachen. Hierdurch wird die Anfärbbarkeit mit Anionfarbstoffen ausgeprägt verbessert.

Gegenüber den Verbindungen der Formel (I) weisen die erfindungsgemäßen Reaktionsprodukte eine ausgeprägt erhöhte Stabilisierungswirkung auf Anionfarbstoffe und insbesondere substantive Farbstoffe auf. Ganz überraschend und außergewöhnlich ist jedoch die Wirkung der erfindungsgemäßen Verbindungen bei Beendigungsvorgängen, die nach dem Färben und Bedrucken mit Reaktivfarbstoffen folgen.

Beendigungs- und Waschverfahren unter Verwendung von Verbindungen der Formel (I) zeichnen sich durch eine intensive Waschwirkung aus, wobei keinerlei Änderungen an den bestehenden Vorrichtungen erforderlich sind. Ferner werden die Lichtechtheit der Ausfärbungen und Drucke in keiner Weise nachteilig beeinflußt. Die Verwendung dieser kationaktiven Polyelektrolyte führt zu einer erheblichen Verkürzung des Waschvorgangs und damit zu einer Einsparung von Wasser, Energie und Lohnkosten.

In wässerigen Lösungen der erfindungsgemäßen kationaktiven Polyelektrolyte kommt es zu einer intensiven Farbstoffdesorption und zur Bildung von Komplexverbindungen mit den Farbstoffen. Diese Komplexe besitzen keine Affinität gegenüber Cellulosefasern und werden auf ihnen nicht sorbiert.

Bei Drucken und Textilmustern, bei denen ein weißer oder heller Grund an einen dunklen Grund angrenzt, kann so beim Waschen ein Ausbluten des ausgewaschenen Farbstoffs in den hellen Grund verhindert werden. Die erfindungsgemäßen Polyelektrolyte können nicht nur technisch-industriell, sondern auch im Haushalt beim Waschen von Textilartikeln mit geringerer Waschechtheit ausgenutzt werden.

Das erfindungsgemäße Verfahren kann ferner auch bei der Reparatur von Textilwaren, bei denen es zu einem Ausbluten des nichtfixierten Farbstoffs gekommen ist, ausgenutzt werden.

Je nach dem Typ der eingesetzten Vorrichtungen beträgt die Konzentration der kationaktiven Polyelektrolyte im Wasserbad 0,1 bis 20 g/l; die Textilmaterialien werden in diesem Bad bei einer Temperatur von 15 bis 100 °C 10 s bis 60 min gewaschen. Die Waschwirkung erhöht sich mit der Badtemperatur; der geeignetste Temperaturbereich ist von 80 bis 100 °C.

Der Effekt der erfindungsgemäßen Polyelektrolyte beim Färben mit Substantivfarbstoffen ist im Vergleich mit den Reaktivfarbstoffen unterschiedlich. Die Polyelektrolyte wirken ähnlich wie die bekannten, beispielsweise von Dicyandiamid abgeleiteten Fixierungsmittel. Sie erhöhen also die Farbechtheit. Im Unterschied zu den bekannten Verbindungen vermindern sie jedoch nicht die Lichtechtheit, was einen weiteren Vorteil darstellt.

Infolge der geeigneten Lage der —NH — und —OH Gruppen sind die kationaktiven Polyelektrolyte imstande, Metallkationen zu binden, so daß mit ihnen fixierte Ausfärbungen mit wässerigen Lösungen von Salzen eines geeigneten Metalls behandelt werden können. Dadurch erhöht sich noch die Naßechtheit und auch die Lichtechtheit. Dabei sind wasserlösliche Salze von $Ca^{2+}$, $Mg^{2+}$, $Zn^{2+}$, $Cu^{2+}$, $Fe^{2+}$, $Co^{2+}$ und $Ni^{2+}$ vorteilhaft geeignet. Die wässerigen Lösungen für derartige Behandlungen enthalten 0,1 bis 20 g/l eines zweiwertigen oder dreiwertigen Kations. Die Salze können gleichzeitig mit dem kationaktiven Polyelektrolyt oder auch anschließend verwendet werden.

Die Herstellung erfindungsgemäßer kationaktiver Polyelektrolyte und ihre Verwendung zur Textilveredlung ist in den folgenden Beispielen näher erläutert.

## Beispiel 1

In einem mit Rückflußkühler, Rührwerk, Thermometer und Dosierungsscheidetrichter versehenen gläsernen Vierhalskolben wurden 318 g einer 83-%igen wässerigen Lösung von 1,3-Bis(3-chlor-2-hydroxypropyl)imidazoliumsulfat vorgelegt. Die Temperatur wurde auf 25 °C gebracht, worauf innerhalb von 60 min 120 g einer 50-%igen Ethylendiaminlösung unter stetigem Rühren in den Kolben dosiert wurden. Dabei begann die Temperatur des Reaktionsgemisches spontan zu steigen. Durch Kühlung und über die Geschwindigkeit der Ethylendiamin-Zugabe wurde die Temperatur im Bereich von 65 bis 70 °C gehalten. Nach vollständiger Zugabe des Ethylendiamins wurde das Reaktionsgemisch bei der gleichen Temperatur noch weitere 30 min reagieren gelassen. Nach dem Abkühlen lag das Reaktionsgemisch als gelbbraune viskose Flüssigkeit mit einer Dichte von 1,43 g/ml vor, die in jedem Verhältnis mit Wasser mischbar war.

## Beispiel 2

In die Glasapparatur von Beispiel 1 wurden 450 g einer 71-%igen wässerigen Lösung von 1,4-Bis(3-chlor-2-hydroxypropyl)-1-methylpiperaziniumchlorid eingegossen. Entsprechend dem in Beispiel 1 beschriebenen Verfahren wurden 120 g 50-%iges Ethylendiamin zudosiert. Das entstandene Reaktionsgemisch war eine viskose Flüssigkeit von gelbbrauner Farbe und einer Dichte von 1,35 g/ml.

Anstelle von 1,4-Bis(3-chlor-2-hydroxypropyl)-1-methylpiperaziniumchlorid wurden ferner
490 g 1,3-Bis(3-chlor-2-hydroxypropyl)-pyrimidindiyliumsulfat (78 %),
432 g 1,4-Bis(3-chlor-2-hydroxypropyl)-pyrazindiyliumacetat (84 %) sowie
240 g 1,3-Bis(2,3-epoxypropyl)-imidazoliumchlorid (75 %) verwendet.

## Beispiel 3

In einem mit Rührwerk, Thermometer und Rückflußkühler versehenen emaillierten Reaktor wurden 140 kg einer wässerigen Lösung von 1,3-Bis(3-chlor-2-hydroxypropyl)-imidazoliumsulfat (Gehalt an aktiver Substanz 83 %) vorgelegt. Bei einer Temperatur von 25 °C wurde dann eine 50-%ige wässerige Lösung von Diethylentriamin zudosiert. Die Zugabegeschwindigkeit wurde dabei so gewählt, daß die Temperatur des Reaktionsgemisches 70 °C nicht überstieg.

Nach Zugabe einer Gesamtmenge von 80 kg der 50-%igen Lösung des Diethylentriamins wurde das Reaktionsgemisch noch weitere 45 min bei 70 °C reagieren gelassen und dann abgekühlt. Die entstandene viskose braune Flüssigkeit wies eine Dichte von 1,47 g/ml auf.

Weitere erfindungsgemäße Polyelektrolyte wurden hergestellt, indem anstelle von 80 kg Diethylentriamin (50 %) 110 kg Hexamethylendiamin (50 %), 70 kg N,N'-Dimethylethylendiamin (50 %) bzw. 116 kg Triethylentetramin (50 %) verwendet wurden.

Beispiel 4

Merzerisiertes Baumwollgarn, das zu einer Farbtiefe von 2 % mit Direktorange C.I. 39 ausgefärbt worden war, wurde bei einer Temperatur von 30 °C in einer Flotte, die 1 g/l des nach Beispiel 1 hergestellten Polyelektrolyts enthielt, bei einem Flottenverhältnis von 1 : 20 10 min behandelt. Danach folgten Abschleudern und Trocknen. Die Ausfärbung wies gute Wasser-, Schweiß- und Waschechtheit bei 40 °C auf.

Beispiel 5

Das Baumwollgarn gemäß Beispiel 4 wurde in einer Flotte, die 5 g/l des nach Beispiel 1 hergestellten Polyelektrolyts und 2 g/l Natriumhydroxid enthielt, bei 50 °C und einem Flottenverhältnis von 1 : 20 30 min behandelt. Nach Waschen und Neutralisierung wurde das Garn getrocknet. Die Ausfärbung wies vorzügliche Wasser-, Schweiß- und Waschechtheit bei 95 °C auf. Es handelte sich also hierbei um eine Ausfärbung mit einem höheren Echtheitsniveau als beim Beendigungsverfahren nach Beispiel 4.

Beispiel 6

Eine Baumwollwirkware wurde auf der Färbehaspel durch einen üblichen Kochprozeß und Bleichen vorbehandelt. Danach wurde bei einem Flottenverhältnis von 1 : 20 in folgender Flotte behandelt :

| | |
|---|---|
| Polyelektrolyt 2 von Tabelle 1 | 15 g/l, |
| Natriumhydroxid | 6 g/l. |

Die Behandlungstemperatur betrug 55 °C, die Behandlungszeit war 50 min. Die Wirkware wurde nach dem Waschen nach der üblichen Verfahrensweise mit Reaktivblau C.I. 4 gefärbt. Im Vergleich mit dem konventionellen Färben wurde doppelte Farbstoffausbeute erzielt.

Beispiel 7

Ein Gewebe aus Polyester- und Cellulosefasern (67/33 %) wurde am Foulard mit einer Flotte folgender Zusammensetzung imprägniert :

| | |
|---|---|
| Dispersblau C.I. 73 | 10 g/l, |
| Reaktivblau C.I. 5 | 3 g/l, |
| Natriumalginat | 2 g/l. |

Nach Trocknen bei 100 °C und 50 s Thermosolation bei 195 °C wurde das Gewebe mit einer Flotte folgender Zusammensetzung imprägniert :

| | |
|---|---|
| Polyelektrolyt 1 von Tabelle 1 | 60 g/l, |
| Natriumhydroxid | 20 g/l. |

Nach dem Durchgang durch den Foulard und Abquetschen auf 70 % wurde das Gewebe 10 h unter stetiger Drehung bei Raumtemperatur abgelagert. Im Verlauf der Ablagerung kam es zu einer vollständigen Fixierung des Reaktivfarbstoffs und Vernetzung der Cellulosefasern durch die erfindungsgemäße Verbindung und somit zu einer Verbesserung der bügelfesten Ausrüstung.

Beispiel 8

Ein Baumwollgewebe, das mit sechs Druckfarben bedruckt worden war, welche die Reaktivfarbstoffe

| | |
|---|---|
| A) Reaktivgelb | C.I. 3, |

| | |
|---|---|
| B) Reaktivorange | C.I. 5, |
| C) Reaktivrot | C.I. 24, |
| D) Reaktivblau | C.I. 5, |
| E) Reaktivgrün | C.I. 8 bzw. |
| F) Reaktivschwarz | C.I. 8 sowie |

Verdickungsmittel, Alkali und Antireduzierzusatz enthielten, wurde nach Dampffixierung in einer Breitwaschmaschine mit acht Abteilen gewaschen, wobei in den einzelnen Abteilen folgende Flotten eingesetzt wurden :

1. kaltes Wasser ;
2. 50 °C warmes Wasser ;
3., 4., 5. kationaktiver Polyelektrolyt 1 von Tabelle 1 (2 g/l), Flottentemperatur 90 °C ;
6. 80 °C warmes Wasser ;
7. 50 °C warmes Wasser ;
8. kaltes Wasser.

Durch einen Durchgang des Gewebes durch die Waschmaschine mit einer Geschwindigkeit von 70 m/min wurde ein vollständiges Auswaschen des nichtfixierten Farbstoffes erzielt. Der Druck wies hohe Wasser- und Waschechtheit auf. Die Lichtechtheit war ferner nicht beeinträchtigt.

## Beispiel 9

Ein auf X-Spulen aufgewickeltes Baumwollgarn wurde im Färbeapparat auf übliche Weise mit folgenden Farbstoffen gefärbt :

| | | |
|---|---|---|
| Reaktivorange | C.I. 4 | 2,0 % |
| Reaktivrot | C.I. 2 | 3,5 % und |
| Reaktivblau | C.I. 4 | 0,2 %. |

Nach dem Färben wurde das Garn 5 min mit kaltem Wasser und dann noch weitere 5 min mit 50 °C warmem Wasser gewaschen. Dann wurde es in einer wässerigen Flotte behandelt, die 2 g/l des kationaktiven Polyelektrolyts 2 von Tabelle 1 enthielt (Temperatur 90 °C, Dauer 10 min). Dann folgten 5 min Waschen mit 50 °C warmem Wasser und kurzes Spülen mit kaltem Wasser. Die Waschechtheiten dieser sehr tiefen Ausfärbung betrugen 4-5/4-5/4-5, während bei konventionell benndeter Ausfärbung Waschecht- heiten von 4/3/4 erzielt wurden.

## Beispiel 10

Eine Wirkware aus einer Baumwolle-Zellwolle-Mischung wurde in einer hydrodynamischen Färbemaschine in üblicher Weise mit Reaktivrot C.I. 2 (4,5 %) gefärbt.

Nach dem Färben wurde die Wirkware 5 min mit 20 bis 25 °C warmem Wasser und danach 5 min mit 60 °C warmem Wasser gewaschen und anschließend 10 min in einer Flotte mit 2 g/l des kationaktiven Polyelektrolyts 3 von Tabelle 1 bei 90 °C behandelt.

Die Naßechtheiten waren deutlich besser als nach konventioneller Beendigung der Färbung.

## Beispiel 11

Ein am Jigger mit Direktblau C.I. 78 zu einer Farbtiefe von 2 % gefärbtes Baumwollgewebe wurde nach dem Färben mit kaltem Wasser gewaschen und bei 30 °C in einer Flotte behandelt, die 5 g/l des Polyelektrolyts 1 von Tabelle 1 (Flottenverhältnis 1 : 5) enthielt. Die so fixierte Ausfärbung wies ausgezeichnete Wasserechtheit und gute Waschechtheit auf.

## Beispiel 12

Das nach Beispiel 4 ausgefärbte und fixierte Baumwollgewebe wurde noch 20 min bei 60 °C und einem Flottenverhältnis von 1 : 5 in einer Flotte behandelt, die aus kristallinem Kupfer (II)-sulfat (2,5 g/l) und Eisessig (1,0 ml/l) hergestellt worden war.

Hierdurch wurden die Wasch- und Lichtechtheit noch weiter erhöht.

(Siehe Tabellen Seite 9 ff.)

Tabelle 1

| Polyelektrolyt Nr. | Ausgangssubstanzen | |
|---|---|---|
| | $\left[ Y-\dot{M}-\dot{Y} \right]^{k\oplus}$ (I) $\frac{k}{r} X^{r\ominus}$ | $H \left[ \overset{R_3}{N} - (CH_2)_n \right]_s - \overset{R_4}{N} - H$ (II) |
| 1 | 1,3-Bis(3-chlor-2-hydroxypropyl)-imidazoliumsulfat 1 mol | Ethylendiamin 1 mol |
| 2 | 1,4-Bis(3-chlor-2-hydroxypropyl)-pyrazindiyliumchlorid 1 mol | Diethylentriamin 0,8 mol |
| 3 | 1,3-Bis(2,3-epoxypropyl)-imidazoliumchlorid 1 mol | Triethylentetramin 0,6 mol |
| 4 | 1,3-Bis(3-chlor-2-hydroxypropyl)-pyrimidindiyliumsulfat 1 mol | Hexamethylendiamin 1 mol |
| 5 | 1,4-Bis(3-chlor-2-hydroxypropyl)-1-methylpiperaziniumacetat 1 mol | N,N'-Dimethylhexamethylendiamin 1 mol |
| 6 | 1,4-Bis(3-chlor-2-hydroxypropyl)-1,4-diethylpiperaziniumsulfat 1 mol | Ethylendiamin 0,8 mol |

Tabelle 2

| Polyelek-trolyt Nr. | Elementare Struktureinheiten |
|---|---|
| 1 | 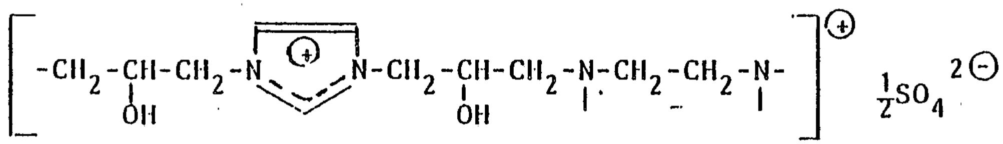 |
| 2 | 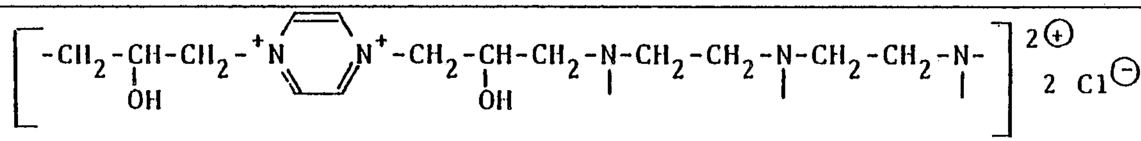 |
| 3 | 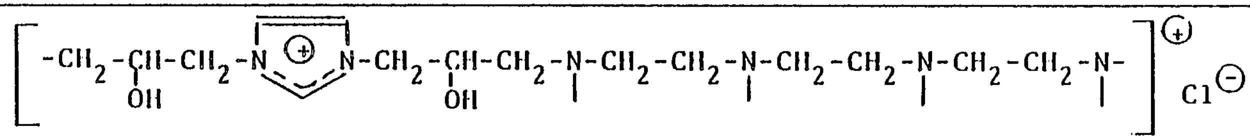 |
| 4 | 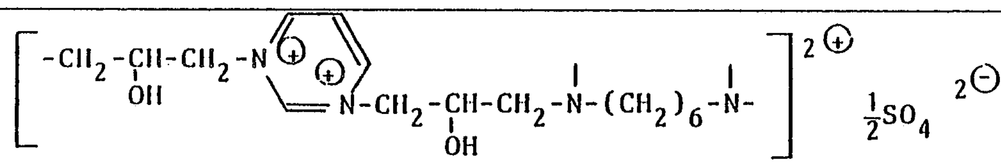 |
| 5 | 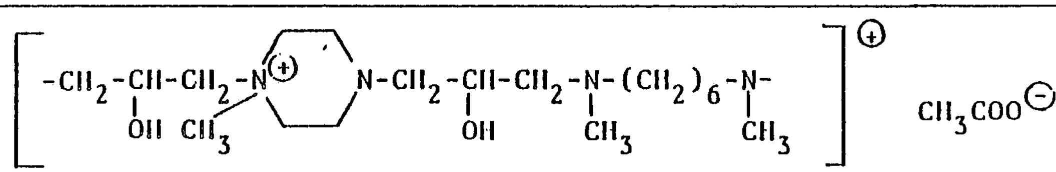 |
| 6 | 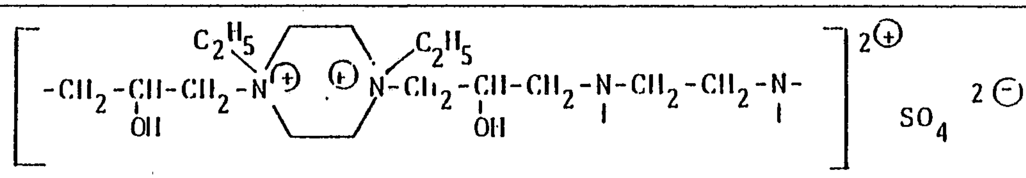 |

**Patentansprüche**

1. Wasserlösliche kationaktive Polyelektrolyte, erhältlich durch Umsetzung einer Verbindung der allgemeinen Formel (I),

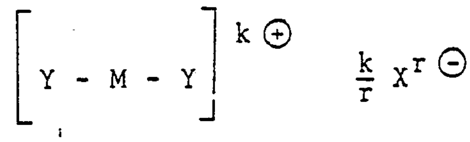 (I)

worin bedeuten :

Y $—CH_2—CH(OH)—CH_2Cl$ oder

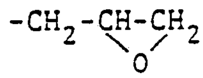 ,

M eines der bifunktionellen heterocyclischen Kationen

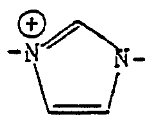 , 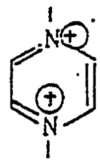 , 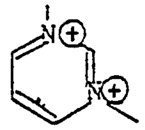 , 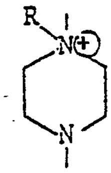

und

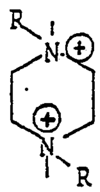 ,

wobei R jeweils H oder $C_{1-4}$-Alkyl darstellt,

X ein Anion einer starken anorganischen oder organischen Säure,

k 1 oder 2

und

r 1, 2 oder 3,

mit einem polyfunktionellen Amin der allgemeinen Formel (II),

$$H—B—H \quad (II)$$

worin B bedeutet :

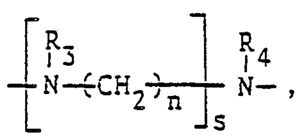 , 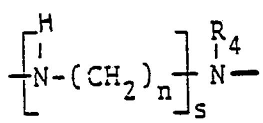

oder

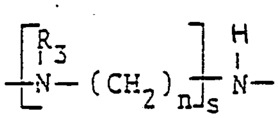

wobei

$R_3$ und $R_4$ unabhängig H oder $C_{1-4}$-Alkyl,

n eine ganze Zahl von 2 bis 10

und

s 1, 2, 3 oder 4 darstellen,

wobei das Molverhältnis (I) : (II) 0,5 bis 10 und die Reaktionstemperatur 10 bis 100 °C betragen.

2. Polyelektrolyte nach Anspruch 1, wobei M ein Imidazolium-Kation ist.

3. Polyelektrolyte nach Anspruch 1 oder 2, wobei die polyfunktionellen Amine der Formel (II) unter Alkylendiaminen, Dialkylentriaminen und Trialkylentetraminen mit $C_{2-6}$-Alkylengruppen ausgewählt sind.

4. Polyelektrolyte nach Anspruch 3, wobei die polyfunktionellen Amine der Formel (II) unter Ethylendiamin, Diethylentriamin, Triethylentetramin, Hexamethylendiamin und N-N'-Dimethylhexamethylendiamin ausgewählt sind.

5. Polyelektrolyte nach einem der Ansprüche 1 bis 4, erhältlich durch Umsetzung der Verbindungen der Formeln (I) und (II) in einem Molverhältnis (I) : (II) von 1 bis 2.

6. Verfahren zur Herstellung der wasserlöslichen kationaktiven Polyelektrolyte nach einem der Ansprüche 1 bis 5, gekennzeichnet durch Umsetzung einer Verbindung der allgemeinen Formel (I),

$$\left[ Y - M - Y \right]^{k\oplus} \quad \frac{k}{r} X^{r\ominus} \qquad (I)$$

worin bedeuten :

Y $-CH_2-CH(OH)-CH_2Cl$ oder

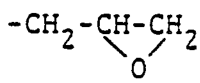 ,

M eines der bifunktionellen heterocyclischen Kationen

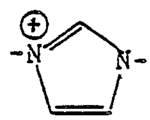 , 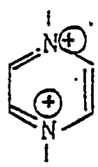 , 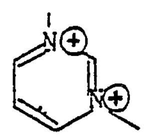 , 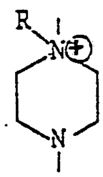

und

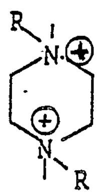 ,

wobei R jeweils H oder $C_{1-4}$-Alkyl darstellt,

X ein Anion einer starken anorganischen oder organischen Säure,

k 1 oder 2

und

r 1, 2 oder 3,

mit einem polyfunktionellen Amin der allgemeinen Formel (II)

$$H-B-H \qquad (II)$$

worin B bedeutet :

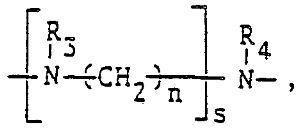 , 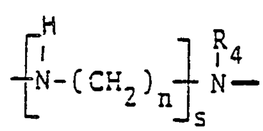

oder

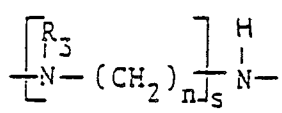

wobei

$R_3$ und $R_4$ unabhängig H oder $C_{1-4}$-Alkyl,

n eine ganze Zahl von 2 bis 10

und

s 1, 2, 3 oder 4 darstellen,

wobei das Molverhältnis (I) : (II) 0,5 bis 10 und die Reaktionstemperatur 10 bis 100 °C betragen.

7. Verfahren nach Anspruch 6, gekennzeichnet durch Verwendung von Verbindungen der Formel (I), in der M ein Imidazolium-Kation ist.

8. Verfahren nach Anspruch 6 oder 7, gekennzeichnet durch Verwendung von Alkylendiaminen, Dialkylentriaminen und/oder Trialkylentetraminen mit $C_{2-6}$-Alkylengruppen als polyfunktionelle Amine der Formel (II).

9. Verfahren nach Anspruch 8, gekennzeichnet durch Verwendung von Ethylendiamin, Diethylentriamin, Triethylentetramin, Hexamethylendiamin und/oder N,N'-Dimethylhexamethylendiamin als polyfunktionelle Amine der Formel (II).

10. Verfahren nach einem der Ansprüche 6 bis 9, gekennzeichnet durch Umsetzung der Verbindungen der Formeln (I) und (II) in einem Molverhältnis (I) : (II) von 1 bis 2.

11. Verwendung der kationischen Polyelektrolyte nach einem der Ansprüche 1 bis 5 zur Beendigung und zum Waschen von Ausfärbungen und/oder Drucken auf Textilmaterialien, die Cellulosefasern enthalten.

12. Verwendung nach Anspruch 11, dadurch gekennzeichnet, daß die gefärbten und/oder bedruckten Textilmaterialien bei Temperaturen von 10 bis 100 °C mit wäßrigen Lösungen behandelt werden, welche die wasserlöslichen kationaktiven Polyelektrolyte in einer Konzentration von 0,1 bis 50 g/l enthalten.

**Claims**

1. Water-soluble cation-active polyelectrolyte which can be obtained by reaction of a compound of the general formula (I)

$$\left[ Y - M - Y \right]^{k\oplus} \quad \frac{k}{r} X^{r\ominus} \qquad (I)$$

wherein : Y is $-CH_2-CH(OH)-CH_2Cl$ or

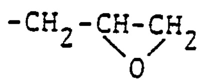 ,

M is one of the bifunctional heterocyclic cations :

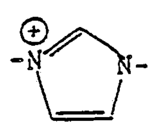 , 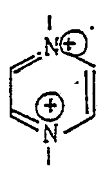 , 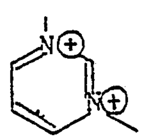 , 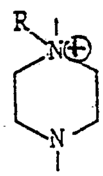

and

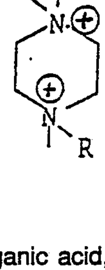 ,

wherein

R respectively denotes H or $C_{1-4}$ alkyl,

X is an anion of a strong inorganic or organic acid,

k is 1 or 2 and

r is 1, 2 or 3

with a polyfunctional amine of the general formula (II)

$$H—B—H \quad (II)$$

wherein B is :

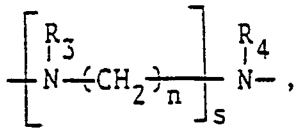, 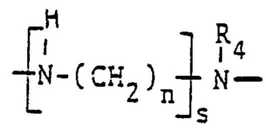

or

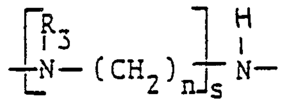

wherein

$R_3$ and $R_4$ are independently H or $C_{1-4}$ alkyl,
n is an integer of from 2 to 10, and
s is 1, 2, 3 or 4,

wherein the molar ratio (I) : (II) is 0.5 to 10 and the reaction temperature is 10 to 100 °C.

2. Polyelectrolyte according to claim 1 wherein M is an imidazole cation.

3. Polyelectrolyte according to claim 1 or claim 2 wherein the polyfunctional amines of formula (II) are selected from alkylene diamines, dialkylene triamines and trialkylene tetramines with $C_{2-6}$ alkylene groups.

4. Polyelectrolyte according to claim 3 wherein the polyfunctional amines of formula (II) are selected from ethylene diamine, diethylene triamine, triethylene tetramine, hexamethylene diamine and N,N'-dimethyl hexamethylene diamine.

5. Polyelectrolyte according to one of claims 1 to 4 which can be obtained by reaction of the compounds of formulae (I) and (II) in a molar ratio (I) : (II) of 1 to 2.

6. A process for the production of the water-soluble cationactive polyelectrolyte according to one of claims 1 to 5, characterised by reaction of a compound of the general formula (I)

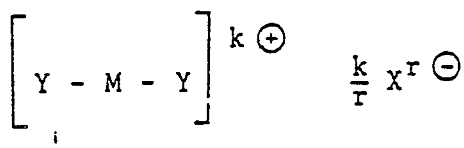 (I)

wherein : Y is $—CH_2—CH(OH)—CH_2Cl$ or

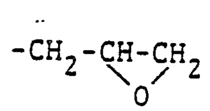,

M is one of the bifunctional heterocyclic cations :

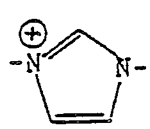, 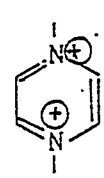, 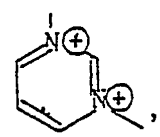, 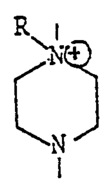

and

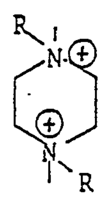,

wherein R respectively denotes H or $C_{1-4}$ alkyl,
X is an anion of a strong inorganic or organic acid,
k is 1 or 2 and
r is 1, 2 or 3
with a polyfunctional amine of the general formula (II)

$$H—B—H \quad (II)$$

wherein B is :

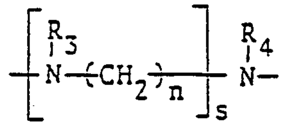
,
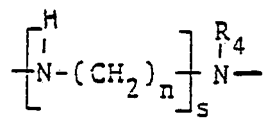

or

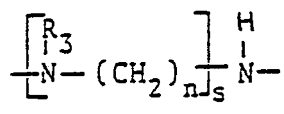

wherein
$R_3$ and $R_4$ are independently H or $C_{1-4}$ alkyl,
n is an integer of from 2 to 10, and
s is 1, 2, 3 or 4,
wherein the molar ratio (I) : (II) is 0.5 to 10 and the reaction temperature is 10 to 100 °C.

7. A process according to claim 6 characterised by the use of compounds of formula (I) in which M is an imidazole cation.

8. A process according to claim 6 or claim 7 characterised by the use of alkylene diamines, dialkylene triamines, and/or trialkylene tetramines with $C_{2-6}$ alkylene groups as polyfunctional amines of the formula (II).

9. A process according to claim 8 characterised by the use of ethylene diamine, diethylene triamine, triethylene tetramine, hexamethylene diamine and/or N,N′-dimethylhexamethylenediamine as polyfunctional amines of the formula (II).

10. A process according to one of claims 6 to 9 characterised by reaction of the compounds of the formulae (I) and (II) in a molar ratio (I) : (II) of 1 to 2.

11. Use of the cationic polyelectrolyte according to one of claims 1 to 5 for ending and washing dyeing operations and/or printing operations on texı 'e materials which contain cellulose fibres.

12. Use according to claim 11 characterised 'n that the dyed and/or printed textile materials are treated at temperatures of from 10 to 100 °C with aqueous solutions which contain the water-soluble cation — active polyelectrolytes in a concentration of from 0.1 to 50 g/l.

## Revendications

1. Polyélectrolytes cationactifs solubles dans l'eau, susceptibles d'être obtenus par réaction d'un composé de formule générale (I),

$$\left[ Y - M - Y \right]^{k\oplus} \quad \frac{k}{r} X^{r\ominus} \qquad (I)$$

dans laquelle signifient :
Y $—CH_2—CH(OH)—CH_2Cl$ ou

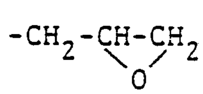
,

M un des cations bifonctionnels hétérocycliques

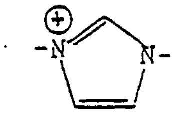 , 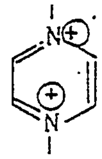 , 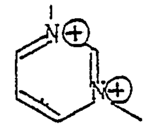 , 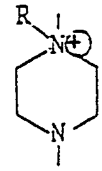

et

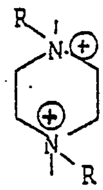 ,

R étant H ou $C_{1-4}$-alkyl,
X un anion d'un acide minéral ou organique fort,
k 1 ou 2
et
r 1, 2 ou 3, avec une amine polyfonctionnelle de formule générale (II)

$$H—B—H \qquad (II)$$

dans laquelle B signifie :

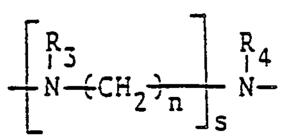 , 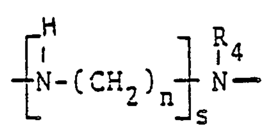

ou

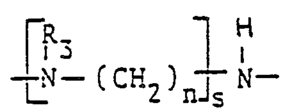

$R_3$ et $R_4$ étant indépendamment H ou $C_{1-4}$-alkyl,
n un nombre entier de 2 à 10
et
s 1, 2, 3 ou 4,
le rapport molaire (I) : (II) étant de 0,5 à 10 et la température de réaction de 10 à 100 °C.

2. Polyélectrolytes selon la revendication 1, dans laquelle M est un cation d'imidazolium.

3. Polyélectrolytes selon la revendication 1 ou 2, dans laquelle les amines polyfonctionnelles de formule (II) sont choisies parmi les alkylènediamines, les dialkylènetriamines et les trialkylènetetramines avec des groupes d'alkylène de 2 à 6 atomes de carbone.

4. Polyélectrolytes selon la revendication 3, dans laquelle les amines polyfonctionnelles de formule (II) sont choisies parmi l'éthylènediamine, la diéthylènetriamine, la triéthylènetetramine, l'hexaméthylènediamine et la N,N'-diméthylhexamethylènediamine.

5. Polyélectrolytes selon une des revendications 1 à 4, susceptibles d'être obtenus par réaction de composés de formule (I) et (II) dans un rapport molaire (I) : (II) de 1 à 2.

6. Procédé pour la préparation de polyélectrolytes cationactifs solubles dans l'eau selon une des revendications 1 à 5, caractérisé par réaction d'un composé de formule générale (I),

$$\left[ Y - M - Y \right]^{k\oplus} \quad \frac{k}{r} X^{r\ominus} \qquad (I)$$

dans laquelle signifient
Y $—CH_2—CH(OH)—CH_2Cl$ ou

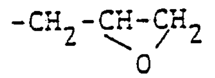 ,

M un des cations bifonctionnels hétérocycliques

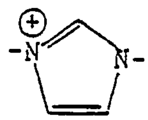 , 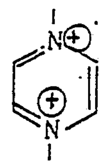 , 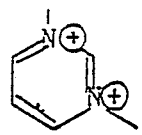 , 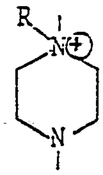

et

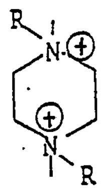 ,

R étant H ou $C_{1-4}$-alkyl,
X un anion d'un acide minéral ou organique fort,
k 1 ou 2
et
r 1, 2 ou 3,
avec une amine polyfonctionnelle de formule générale (II)

H—B—H (II)

dans laquelle B signifie :

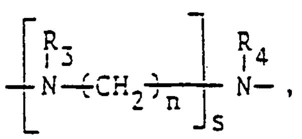 , 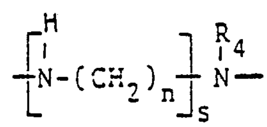

ou

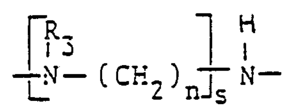

$R_3$ et $R_4$ étant indépendamment H ou $C_{1-4}$-alkyl,
n un nombre entier de 2 à 10
et
s 1, 2, 3 ou 4,
le rapport molaire (I) : (II) étant de 0,5 à 10 et la température de réaction de 10 à 100 °C.

7. Procédé selon la revendication 6, caractérisé par l'utilisation de composés de formule (I), dans laquelle M est un cation d'imidazolium.

8. Procédé selon la revendication 6 ou 7, caractérisé par l'utilisation d'alkylènediamines, de dialkylènetriamines et/ou de trialkylènetetramines avec des groupes d'alkylène de 2 à 6 atomes de carbone en tant qu'amines polyfonctionnelles de formule (II).

9. Procédé selon la revendication 8, caractérisé par l'utilisation d'éthylènediamine, de diéthylènediamine, triéthylènetetramine, hexaméthylènediamine et/ou N,N'-diméthylhexaméthylènediamine en tant qu'amines polyfonctionnelles de formule (II).

10. Procédé selon une des revendications 6 à 9, caractérisé par la réaction de composés de formule (I) et (II) dans un rapport molaire (I) : (II) de 1 à 2.

11. Utilisation des polyélectrolytes cationactifs selon une des revendications 1 à 5 pour terminer ou laver la dernière teinture et/ou les impressions d'un matériel textil contenant des fibres de cellulose.

12. Utilisation selon la revendication 11, caractérisée en ce que le matériel textil coloré et/ou imprimé est traité à une température de 10 à 100 °C avec des solutions aqueuses contenant les polyélectrolytes cationactifs solubles dans l'eau dans une concentration de 0,1 à 50 g/l.